# EUROPEAN PATENT APPLICATION

(11) **EP 1 865 321 A1**
(43) Date of publication of application: **12.12.2007**
(21) Application number: 06730208.3
(22) Date of filing: 28.03.2006
(51) Int. Cl.: G01N 33/53, C12N 15/09, C12Q 1/68, G01N 37/00

(54) **DISPLAY METHOD OF MEASUREMENT INFORMATION OF BIOLOGICALLY RELEVANT MATERIAL**

(30) Priority: 30.03.2005 JP 2005097893
(71) Applicant: Olympus Corporation, Tokyo 151-0072 (JP)
(72) Inventor: SAIDA, Yuko, Mito-shi, Ibaraki 3100851 (JP); SAKAMOTO, Hiroko, Shinagawa-ku, Tokyo 1410022 (JP); SATOH, Takatomo, Hino-shi, Tokyo 1910065 (JP); OKAZAKI, Tomoko, Hachioji-shi, Tokyo 1930831 (JP); SANUKI, Hiromi, Yokohama-shi, Kanagawa 2270036 (JP)
(74) Representative: von Hellfeld, Axel
(86) International application number: PCT/JP2006/306260
(87) International publication number: WO 2006/106661

(57) **Abstract**

A display method of measurement information of a biologically relevant material according to the invention displays measurement information on a computer screen when measuring the amount of a biologically relevant material of a test sample or the amount ratio of a biologically relevant material among a plurality of test samples with a microarray.

## Description

### TECHNICAL FIELD

The present invention relates to a display method of measurement information such as a measured object, a measurement condition, an image of a detection result, numerical data and a comparison result by detecting a biochemical reaction of a solid-phase support with solid-phased probes to detect a biologically relevant material.
Priority is claimed on Japanese Patent Application No. 2005-97893, filed March 30, 2005, the content of which is incorporated herein by reference.

### BACKGROUND ART

A microarray has been known as an example of a solid phase support of which a probe to detect a biologically relevant material is solid-phased. For example, DNA probes which are spotted on the microarray are mixed with a fluorescently labeled nucleic acid fluid originating from a cell to measure the amount of nucleic acid extracted from a cell to be compared, thereby detecting the intensity of fluorescence. As the amount of the nucleic acid is measured by using the intensity of fluorescence, it is possible to examine the expression level of a gene, the presence of a particular gene in a genome, and the gene mutation.

Thus, it is critical to measure the amount ratio of nucleic acid and display a measurement result without difficulty. The appropriate display of the measurement result may improve precision in analyzing the measurement result with the microarray and save time in analyzing the measurement result. An example of the display means includes a three dimensional graph in which an axis X refers to a set of test samples, an axis Y is a microarray set and an axis Z presents the intensity of fluorescence (Patent Document 1).
Patent Document 1: Japanese Unexamined Patent Application, First Publication (JP-A) No. 2001-41892

However, according to the invention of Patent Document 1, it is hard to measure the predisposition of the analysis result if the number of analyzed genes is limited, i.e., if a microarray having the small number of probe spots is analyzed. It is difficult to determine how small the small data is compared to the large data at a glance, which may not be an appropriate display method.
As described above, a display method which fully provides an analysis result of a microarray so that a user can identify the predisposition of the analysis result simultaneously has yet to be provided.

### DISCLOSURE OF INVENTION

Accordingly, it is an aspect of the invention to provide a display method which displays information easily to analyze a microarray and determines an analysis result at a glance even if a microarray having the limited number of genes is used.

The invention provides a display method of measurement information of a biologically relevant material which measures the amount of biologically relevant material of a test sample or the amount ratio of the biologically relevant material among a plurality of test samples, by using a microarray, and displays the measurement information on a computer screen.

In the invention, "biologically relevant material" includes various materials which exist or originate from a living body (animal cells, plant cells and microorganism cells, and viruses that proliferate only if being parasitic on those cells), and includes natural, artificially composite materials (e.g. genetically artificial), etc. For example, the biologically relevant material may include nucleic acids such as DNA, cDNA and RNA, proteins such as several types of hormones, a tumor marker, an enzyme, an antibody and an antigen; and complexes such as PNA.
In the invention, "shade" refers to color gradation and color intensity of a carrier or a medium, and may be recognized as differences of optical properties. The difference may be recognized by both a device and the human eye.
"Measurement information" includes overall information such as bibliographic facts including a measured object, a position of a probe spot, a serial number of a microarray, name of experimenter, experiment data and place, a type of a test sample; a measurement condition including a measuring device, a measurement method, a type of an optical filter, a measurement temperature, pH, measurement time; a measurement result such as signal intensity data; and an analysis result of the measurement result such as a data normalization method, the calculation of an amount of the biologically relevant material and an amount ratio of the biologically relevant material among the plurality of test samples.

The display method of the measurement information of the biologically relevant material according to the present invention may have the following aspects (1) to (5).
(1) A replicate image which illustrates a layout of a probe spot in the microarray is displayed as the measurement information.
According to the display method, the amount of the biologically relevant material or the amount ratio of the biologically relevant material among the plurality of test samples may be represented with color gradation or the types of color tones. The position of the probe spot on the microarray or the normalized, biologically relevant material, e.g. the type of a normalized gene, may be displayed to be easily recognized. Further, information on the bibliographic facts may be displayed. Thus, necessary information may be displayed rapidly, sufficiently, moderately and easily. The normalized gene may include a gene (internal control gene) which is included in the test sample, and a gene (external control gene) which is not included in the test sample.

(2) The amount of the biologically relevant material of the test sample which is measured by using the microarray is normalized to compare the amount of the biologically relevant material in the each test sample.
As the amount of the biologically relevant material is normalized and displayed, a bias of data due to differences in the fabricated test samples may be normalized. The display method of the invention may display the normalized data to be analyzed easily and efficiently. The optimal normalization method is selected depending on the type of data, thereby analyzing the data more precisely. According to the present display method, the normalization method may be adjusted from any analysis result displayed on a screen. The optimal normalization method is easily selected to analyze the microarray data by displaying the employed normalization method and normalization coefficients.

From a folder storing the measurement information including the analysis result, the serial number of the microarray, the name of experimenter, the type of the optical filter, the measurement condition such as the measurement temperature, or the amount of the biologically relevant material and the ratio of the biologically relevant materials among the plurality of the test samples may be obtained according to the aspect. Thus, a user may recognize the data analysis result under the predetermined experiment condition simultaneously since the foregoing measurement information is displayed.

(3) A bar graph illustrates the amount ratio of the biologically relevant material of two test samples to be compared with each other.
According to the present display method, the amount ratio of the biologically relevant material is represented with the bar graph. The different amount ratios of the biologically relevant material may be identified by varying the gradation of the bar graph according to the ratio. Then, a user may recognize whether the amount of the biologically relevant material is changed, based on the gradation of the bar graph. Thus, the comparison result is displayed to be viewed easily at a glance.

(4) The relationship between the position of the probe spot and the amount ratio or signal intensity of the biologically relevant material among the two test samples to be compared, with respect to the plurality of probe spots on the microarray, is displayed with a polygonal line graph or a plot.
According to the present display method, changes in the amount ratio or the signal intensity of the biologically relevant material are displayed easily with the polygonal line graph or the plot.

(5) An image which is a combination of images generated under optimal signal detecting conditions for the each probe spot is displayed.
Some probe spots on the microarray are bright while others thereon are dark. Since images which are obtained under the same signal detecting condition are too dark or too bright, the state of the respective probe spots are not recognized precisely. According to the present display method, however, the image which is a combination of the images generated under the optimal signal detecting condition to the respective probe spots is displayed. Thus, the overall probe spots may be analyzed under optimal conditions.
In consideration of the number of graphs that can be displayed on a monitor and the number of graphs recognizeable by a person at a time, the present invention may be applicable to the analysis of 500 items or less (in case of the microarray, the number of analyzed items corresponds to the number of analyzed probe spots. That is, two analyzed items correspond to two equivalent probe spots.).

According to the present invention, data or information required for the analysis are displayed through a plurality of display methods, so that a user may view the data or the information and analyze the microarray having a limited number of genes without difficulty. As the data or the information analysis is implemented without difficulty through the present display method, time analyzing the data is saved and the analysis precision may be improved.

### BRIEF DESCRIPTION OF THE DRAWINGS

FIG 1 illustrates a display method according to an exemplary embodiment of the present invention.
FIG 2 illustrates an example of a replicate image of the display method according to the exemplary embodiment of the present invention.
FIG 3 illustrates an adjustment window which is displayed on the replicate image of the display method according to the exemplary embodiment of the present invention.
FIG 4 illustrates an example of a table according to the exemplary embodiment of the present invention.
FIG 5 illustrates an adjustment window which is displayed on the table according to the exemplary embodiment of the present invention.
FIG 6 illustrates an adjustment window which is displayed on a distribution diagram according to the exemplary embodiment of the present invention.
FIG 7 illustrates a folder and a normalization method used for the analysis and normalization coefficients according to the exemplary embodiment of the present invention.
FIG 8 illustrates measurement information according to the exemplary embodiment of the present invention.
FIG 9 is a ratio diagram illustrating the exemplary embodiment of the present invention.
FIG 10 illustrates an adjustment window which is displayed on the ratio diagram according to the exemplary embodiment of the present invention.
FIG 11 illustrates a main window displaying numerical data alone and another window displaying a graph according to the exemplary embodiment of the present invention.
FIG 12 is a polygonal line graph illustrating the amount ratio of and the signal intensity of a biologically relevant material according to the exemplary embodiment of the present invention.
FIG 13 illustrates an image which is a combination of images generated under an optimal signal detecting condition to respective probe spots on the microarray according to the exemplary embodiment of the present invention.
FIG 14 illustrates an image which is a combination of images generated under an optimal signal detecting conditions to respective probe spots on the microarray according to the exemplary embodiment of the present invention.
FIG 15 illustrates an adjustment window which is displayed on the image which is a combination of the images generated under optimal signal detecting conditions for the respective probe spots on the microarray according to the exemplary embodiment of the present invention.
FIG 16 is a block diagram of a system showing the display method according to the exemplary embodiment of the present invention.
FIG 17 is a schematic flowchart illustrating the display method according to the exemplary embodiment of the present invention.
FIG 18 is a schematic flowchart illustrating the display method according to the exemplary embodiment of the present invention.
FIG 19 is a schematic flowchart illustrating the display method according to the exemplary embodiment of the present invention.
FIG 20 is a schematic flowchart illustrating the display method according to the exemplary embodiment of the present invention.
FIG 21 is a schematic flowchart illustrating the display method according to the exemplary embodiment of the present invention.

### BEST MODE FOR CARRYING OUT THE INVENTION

### First Embodiment

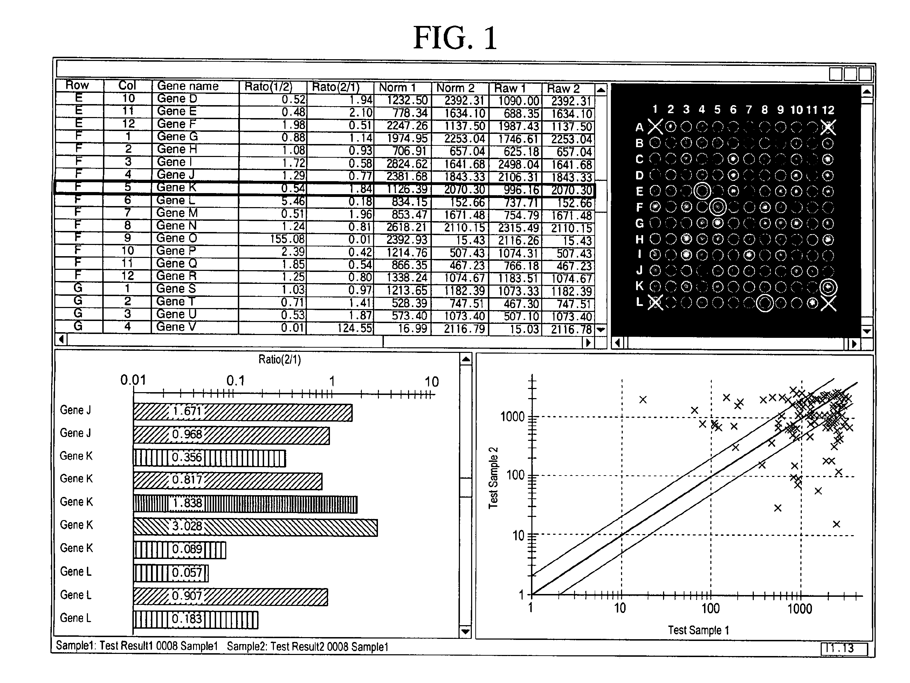

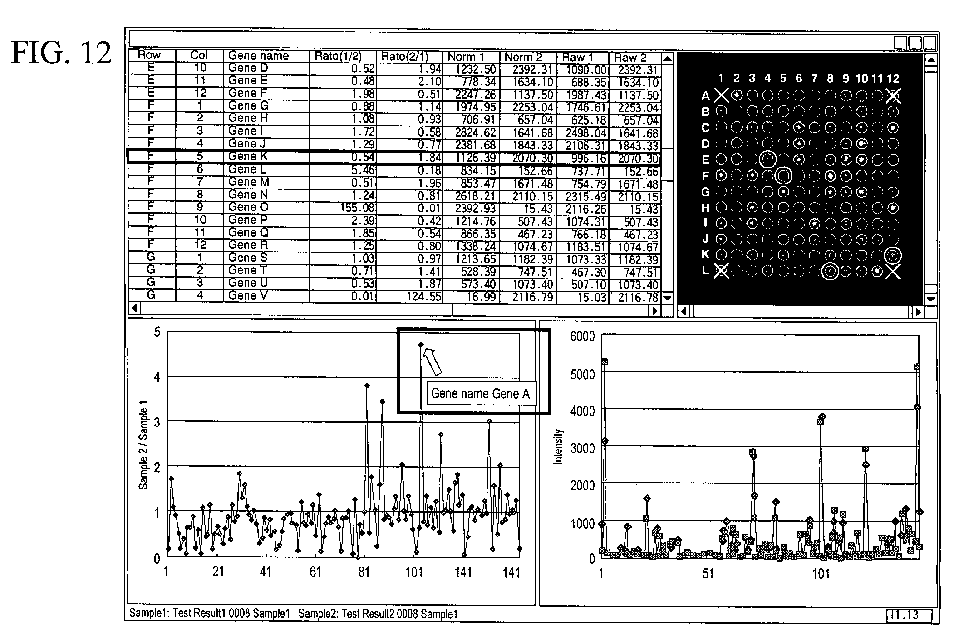

FIG 1 illustrates an example of a display method according to the present invention. As shown therein, numerical data, a replicate image, a bar graph and a distribution diagram are displayed on the same screen. Meanwhile, FIG 12 illustrates another example of the display method according to the present invention. As shown therein, numerical data, a replicate image, a polygonal line graph and a plot are displayed on the same screen. That is, measurement information may be displayed on the same screen through a plurality of different display methods.

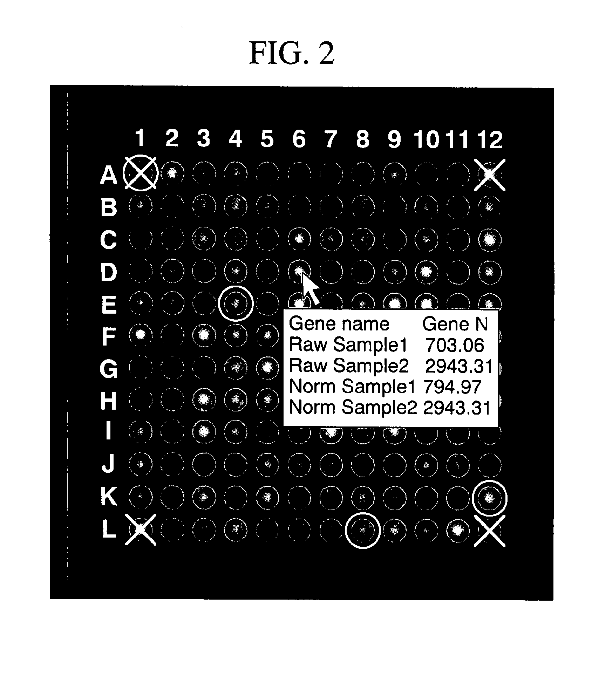

FIG 2 illustrates an example of a replicate image which illustrates a layout of probe spots on a microarray used for analysis. In the replicate image, signal intensity is normalized in 256 gradations. The difference between the maximum signal intensity value and the minimum signal intensity value of the probe spots on a single microarray is significant. Thus, the overall probe spots may be displayed by normalizing the signal intensity of the image. The normalization method includes a method of setting the maximum value or a predetermined signal intensity value of the signal intensity data of the overall measured probe spots, dividing the value by 256 and determining and displaying the signal intensity range displayed as the respective gradations, on the screen. For example, if a single, fairly bright probe spot exists, other probe spots tend to become darker due to the single bright probe spot. In this case, the overall probe spots on the single microarray may be displayed more brightly by normalizing and displaying the gradation from a particular signal intensity. As the signal intensity can be adjusted arbitrarily, the overall probe spots may be displayed regardless of whether the probe spots are bright or dark.

It is possible to select the display method of the data, e.g. displaying the result individually or overlapping and displaying the result. For example, if two test samples are compared with each other, the signal intensity of a sample 1 is displayed in a first gradation, e.g. in red color while the signal intensity of a sample 2 is displayed in a second gradation, e.g. in green color. Then, the overlapped signal intensity is displayed in a gradation mixed with the first and second gradations. As the test samples have different gradations, the data may be analyzed easily by varying the gradations with respect to the signal intensity or the ratio in a probe spot layout.
The data may be analyzed by varying shades with respect to the amount of the biologically relevant material. For example, a low expression level of a gene is displayed in green, a high expression level of a gene is displayed in red and an intermediate expression level of a gene is displayed in yellow. Thus, the overall probe spots are represented in 256 gradations, thereby enabling to analyze the amount and the ratio of the biologically relevant material of the respective probe spots at a glance.

If the signal intensity of the probe spot on the replicate image gradually changes from the center to the circumference, it is similar to a genuine probe spot. That is, the image of each replicate probe spot is displayed on the replicate image in a circle whose color density is higher at the center and gradually becomes lower toward the circumference thereof. Thus, a user may easily get the image as the probe spot layout.
If the position of the probe spot on the microarray is displayed on the replicate image, a user may check the signal intensity of the probe spots depending on positions and check whether the amount ratio of the biologically relevant material changes by viewing only the overall layout.
It is possible to select one of raw data of the signal intensity and normalized data which removes unbiochemical noises therefrom, as the displayed data depending on the analyzed content. For example, the foregoing data may be used to visually compare the overall differences of the raw data or compare the amount of the biologically relevant material. It is preferable to display the raw data of the signal intensity and the normalized data by interchanging them.

It is more preferable that the detailed information on each probe spots is displayed. Such an information display method includes a method of displaying a mark on the probe spots of the replicate image. As shown in FIG 2, a first mark, e.g. a blue circle is displayed on the currently-selected probe spots, a second mark, e.g. X is displayed on the unused probe spots, and a third mark, e.g. a red circle is displayed on the probe spots of the normalized, biologically relevant material, e.g. internal control gene IC (a gene which does not change with respect to any test sample). The probe spots which are not included in the foregoing probe spots do not have a mark thereon. With such marks, by only seeing the replicate images, a user can get the overall image of the layout. The X-marked probe spots may refer to failures in the experiment, and thus are not used, e.g., in calculating the amount ratio of the biologically relevant material.

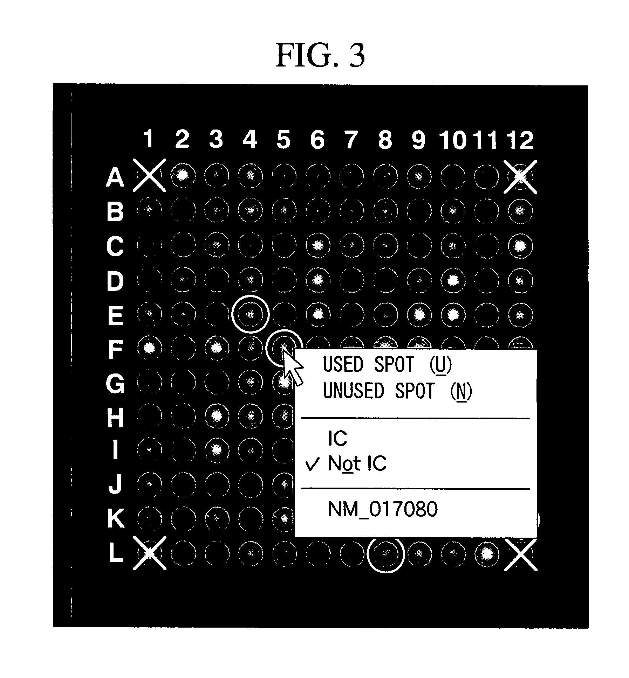

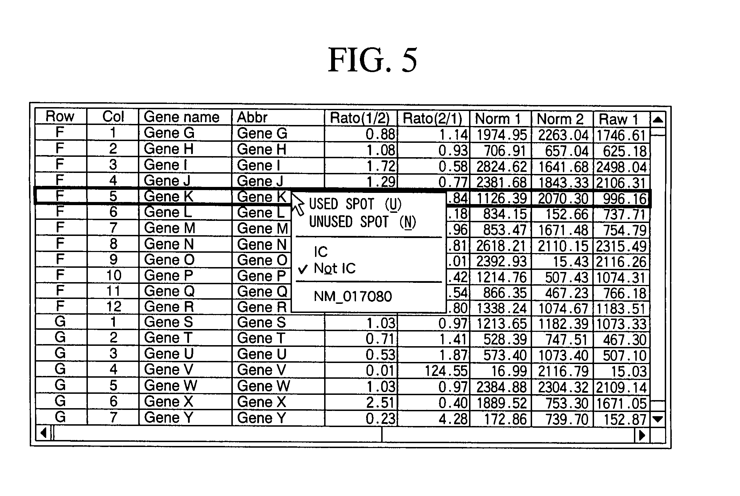

It is preferred that information on the used and unused probe spots may be adjusted on the replicate image. For example, as shown in FIG 3, a user may click the right button on a mouse on the replicate image of the probe spot to display the adjustment window. Then, a user may adjust the currently-selected probe spot whether to be used in the analysis, or whether to be the internal control gene. In this case, the information or the signal intensity of the probe spots also changes. A user may change the information of the probe spots on the replicate image while viewing the relation between the probe spots and the signal intensity. Further, such information may be changed not only on the replicate image but also from a table, a ratio diagram (bar graph) and the distribution diagram as shown in FIGS. 5, 6 and 9. If the information changes, the numerical data, plot and the signal intensity change, too. As the overall displayed information relating to the probe spots are changed, a user may analyze or change the data by using the overall displayed information as well as by viewing the result.

According to another display method, as shown in FIG 2, a measurement information window is displayed if a mouse pointer is placed on the replicate image of the probe spot. The measurement information may include the position of the probe spot, a gene name, raw data of the signal intensity, normalized signal intensity data, etc. With the foregoing display method, a user can view the signal intensity and the position of the probe spots and receive detailed information, thereby analyzing the microarray more rapidly and precisely.
The example of the display method of the replicate image is described above. The display method or the information display items are not limited to those mentioned above, and may vary as long as they are appropriate to analyze the microarray.

### Second Embodiment

When the amount of the biologically relevant material is compared by using test samples obtained from a cell to be compared to analyze the microarray data, unbiochemical noise or bias such as the difference of the RNA amount included in the respective test samples occur in the microarray data. The noises or the bias may be caused by the different fabrications of the test samples. However, it is technically difficult to fabricate test samples not to cause noise or the bias. Thus, it is required to normalize the data (normalization of the bias of the data) between the microarrays from the obtained signal intensity data.

The example of normalization includes a method that the data may be normalized by using an internal control gene if the biologically relevant material is a gene. The internal control gene refers to a gene which does not change with respect to any test sample, e.g. in expression. Thus, if two types of test samples are compared and if the signal intensity value of the internal control gene in the sample 1 is 100 and the signal intensity value of the internal control gene in the sample 2 is 200, the result of the sample 1 can double to normalize the noise or the bias due to the differences in the RNA amount.
As it is important to mark the position (probe spot) of the internal control gene in the analysis result in the course of analysis, the position of the internal control gene may be preferably marked in any part of the analysis result. FIG 2 illustrates an example of displaying the position of the internal control gene on the replicate image. The replicate image of the probe spot which is marked with a red circle represents the internal control gene. As the position of the internal control gene is displayed as above, a user can recognize the arrangement of the internal control gene across the microarray simultaneously.

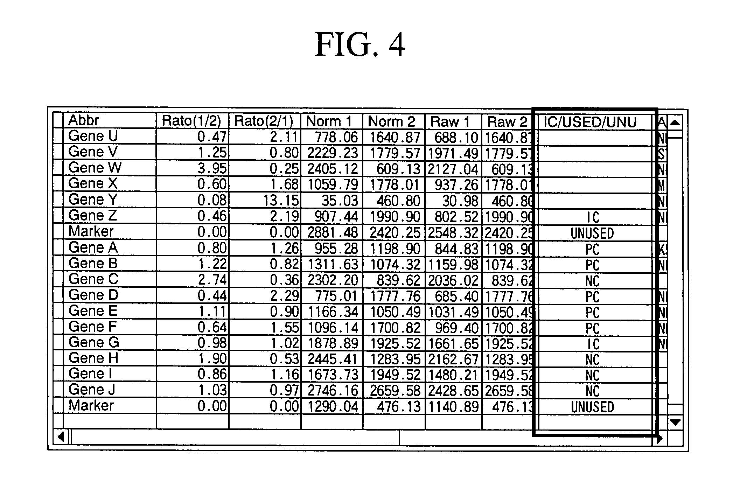

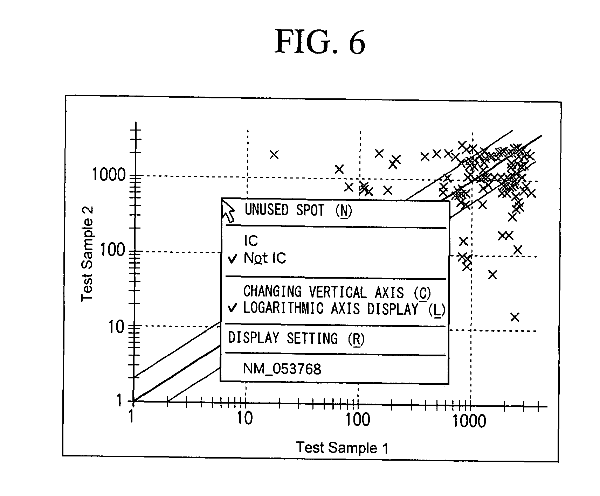

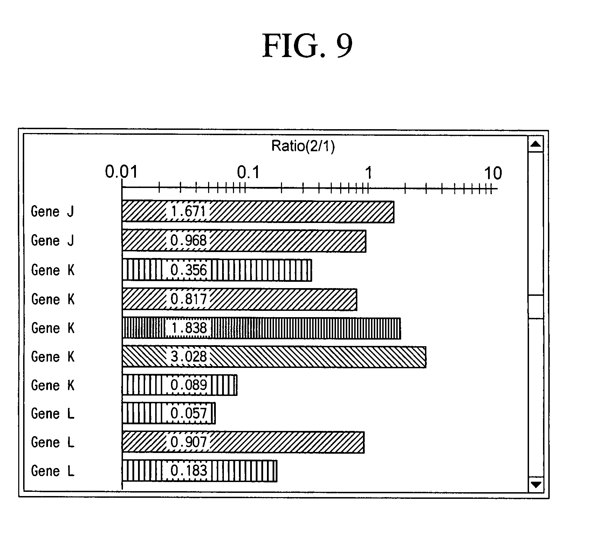

FIG 4 illustrates a table which displays the information on the internal control gene in a raw surrounded by a box as IC (internal control). As the information on the internal control gene is also displayed in the table, a user can analyze the microarray data while checking other information such as gene name, signal intensity, etc. The internal control gene is also displayed in the distribution diagram and the ratio diagram in FIGS. 6 and 9 in a different display color, thereby enabling a user to check the internal control gene from the graphs.
Even if the internal control gene is selected as the unchanged gene at a design stage, it may not be appropriate to use the unchanged gene to normalize the data due to experiment failures. If the selected gene is not the internal control gene in the analysis result, it should be reset. Meanwhile, a gene which was not the internal control gene at the design stage may be reset as the internal control gene. As shown in FIGS. 3, 5, 6 and 10, a user can click the right button of the mouse to display the adjustment window on the probe spots, the plot and the items, and a user can adjust the gene to be an internal control gene or not from any display at any time while analyzing the microarray data. The changed internal control gene can be displayed, e.g. in a different display color.

Another normalization method of the analysis result includes a method of using an average signal intensity value or an intermediate value of the overall probe spots. This method is employed on the assumption that the reaction of the genes arranged on the microarray is not different between the test samples. Thus, the overall detectable genes on the microarray are used to normalize the analysis result. The present method is used if it is difficult to determine the internal control gene.

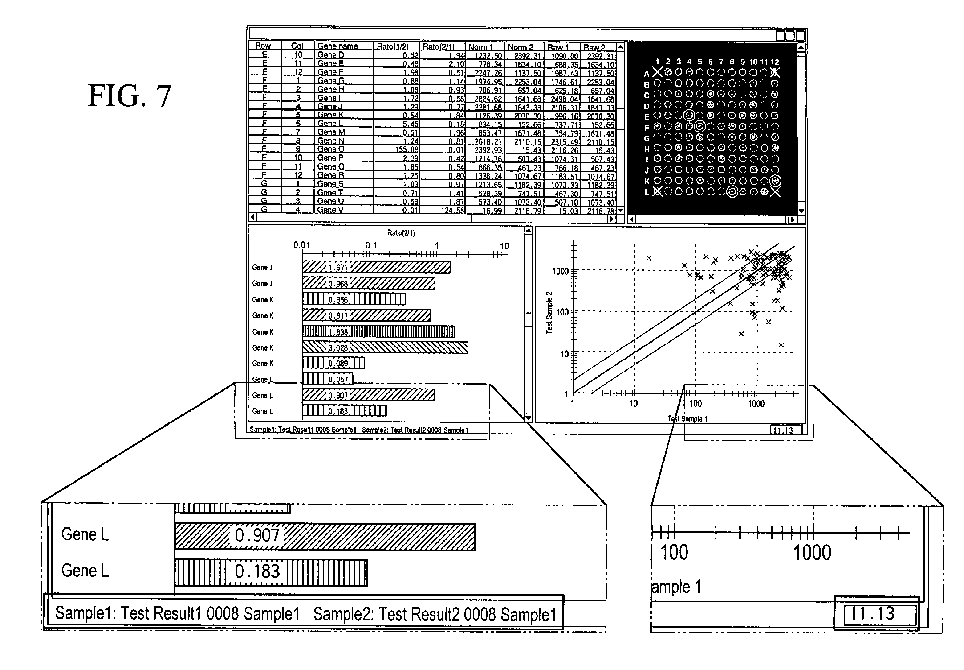

Three normalization methods are described above. A user may select the normalization method while analyzing the microarray data. Preferably, the three normalization methods are used alternately. The current result needs to be changed as a new value to be displayed. If the normalization method is changed, it is important to know which normalization method is used for the currently-displayed result. As shown in FIG 7, "I" (Internal) is displayed when the internal control gene is used, "G" (Global) is displayed when the average value of the overall probe spots is used and "M" (Median) is displayed when the intermediate value of the overall probe spots is used, in the right bottom of the main window. As shown therein, the coefficients which are used to normalize the analysis result are also displayed to select the optimal normalization method.
There are provided three normalization methods above. However, the normalization method is not limited to the three methods described above, and may vary as long it is appropriate to normalize the microarray data. The display and setting methods which are illustrated with the drawings are not limited to those described above, and may vary as long as they can provide and adjust information.

### Third Embodiment

To analyze the microarray data, the compared data, the conditions and the type of the test samples used in the experiment should be considered.

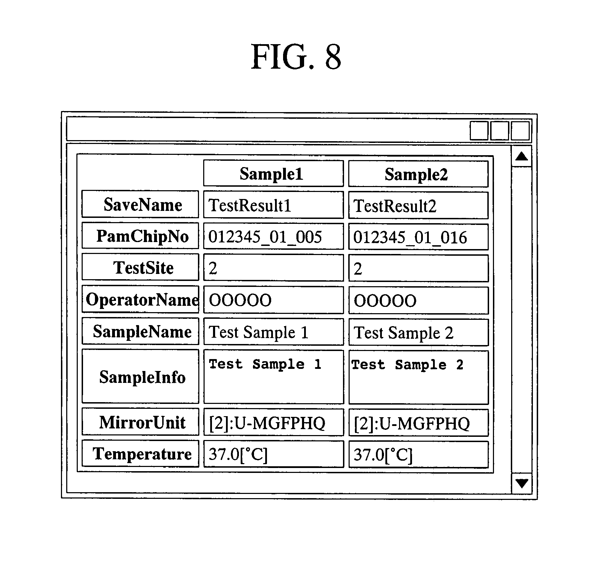

FIG 8 illustrates an example of a measurement information window of respective test samples when the amount of nucleic acid is compared by using the test samples obtained from two cells to be compared. Such a window is displayed more easily than a menu bar or a tool bar, and displayed anytime when the measurement information is required.

For example, the displayed measurement information may include a folder storing the measurement information having the analysis result, the folder name, the type of the test samples, a serial number of the microarray, the name of the experimenter, the type of an exciting and absorbing optical filter used to measure the biologically relevant material, a measurement condition such as the measurement date and place, a measurement temperature, pH and measurement time, the amount of the biologically relevant material and the amount ratio of the biologically relevant material among the plurality of test samples. For example, if the folder storing the measurement information having the analysis result or the folder name is displayed, a user can acknowledge the storing place of the respective data such as the displayed analysis result. Even if a user forgets which data is used, the user can determine the used data by examining the relationship between the original data and the analysis result. Likewise, as the type of the test samples is displayed, a user may recognize the type of the test samples used to obtain the respective analysis data. Therefore, a user can determine whether the type of test samples is correctly used in analysis.
The displayed measurement information is not limited to those described above, and may vary as long as it is necessary to analyze the biologically relevant material including the microarray with respect to various items simultaneously.

### Fourth Embodiment

To analyze a microarray, the amount of a biologically relevant material is compared by using test samples obtained from a cell to be compared. As shown in FIG 9, a bar graph (ratio diagram) is one of the display methods of the comparison result. The vertical axis refers to the type of gene while the horizontal axis represents the amount ratio of nucleic acid. A user may refer to the bar graph and determine whether the amount ratio of nucleic acid rises, falls or remains flat from the length of the bar graph and the figures displayed in the bar graph.

To visualize the comparison result more easily, the bar graph may have shades. For example, the shades of the bar graphs may vary depending on the amount ratio of the biologically relevant material. More specifically, 0.5 and below refers to the declined amount ratio of nucleic acid and the corresponding bar graph is displayed in a first shade, e.g. red color. A range which is larger than 0.5 and smaller than 2 represents the unchanged amount ratio of nucleic acid, and the corresponding bar graph is displayed in a second shade, e.g. yellow, while 2 and above refers to the raised amount ratio of nucleic acid and the corresponding bar graph spot is displayed in a third shade, e.g. green color. The different shades are used to identify the amount ratio of nucleic acid so that a user may recognize the result more easily. If the currently-analyzed probe spot is displayed in a fourth shade, e.g. blue, a user may recognize the displayed result more easily. Instead of the method of varying the colors of the bar graphs displaying the various amount ratios of nucleic acid, a method of illustrating the bar graph thicker with respect to the high expression level of the gene may be used. The thickness of the bar graph may vary or the bar graph may be marked with, e.g. an asterisk and the comparison result may be displayed with the different number of the marks.

The present method can be used in the replicate image in FIG 2, as well as in the bar graph. Like the bar graph, 0.5 and below refers to the declined amount ratio of nucleic acid and the corresponding replicate image of the probe spot is displayed in a first shade, e.g. red. A range which is larger than 0.5 and smaller than 2 represents the unchanged amount ratio of nucleic acid, and the corresponding replicate image of the probe spot is displayed in a second shade, e.g. yellow, while 2 and above refers to the raised amount ratio of nucleic acid and the corresponding replicate image of the probe spot is displayed in a third shade, e.g. green. The present display method provides information such as the arrangement of the probe spots and the gene name together with the amount ratio of nucleic acid so that a user can analyze the microarray data with more information. There may be another method in which the shades are adjusted, e.g. the size of the probe spots on the replicate image is changed or the probe spots on the replicate image are three-dimensional to display the amount ratio of nucleic acid with the height thereof.

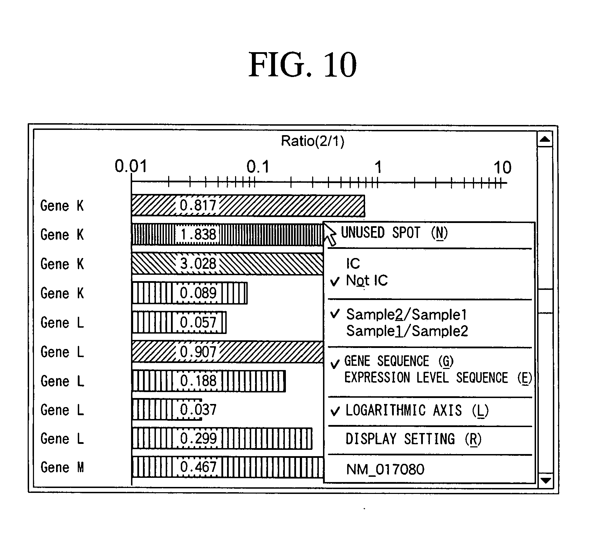

The display state of the bar graph may depend on the type of the biologically relevant material. Thus, the information on the biologically relevant material is sequentially arranged in a vertical or horizontal axis. If the biologically relevant material is a gene, the display sequence may include a gene sequence and an amount ratio sequence of nucleic acid, etc. Preferably, the display sequence is determined according to analysis content. As shown in FIG 10, a selection unit, e.g. the right button of a mouse is clicked on the bar graph to display an adjustment window to change the display sequence. If the adjustment window is open, a user can recognize the current display sequence. The selection unit is not particularly limited as long as it can select the position on the computer screen. From the control point of view, a mouse pointer is most preferable. The selection unit may further include a curser or a touch panel (to be touched by one's finger or a pen).

The range of the amount ratio of the biologically relevant material (in this case the amount of nucleic acid) displayed in the horizontal or vertical axis may be preferably changed. In some cases, a user may desire to check a limited range in more detail depending on the type of analysis even though the data range obtained is not determined. In such a case, as shown in FIG 10, a user may click the right button of the mouse on the bar graph to display the adjustment window, and select a display setting therefrom to set the display range.

The test samples which are selected as a reference (e.g. results from a normal cell) to be compared are changed in the experiment. Preferably, the test samples are selected, referring to the data. For example, if the amount of nucleic acid between two test samples, i.e. between Sample 1 and Sample 2 is compared, Sample 1/Sample 2 or Sample 2/Sample 1 can be calculated and displayed. As shown in FIG 10, a user may click the right button of the mouse on the bar graph to display the adjustment window, and set the display item. When the display item is changed, the item of the horizontal axis in the bar graph is also changed. Thus, a user may recognize the current test sample selected as the reference to analyze the microarray. The reference information can be displayed on the adjustment window.

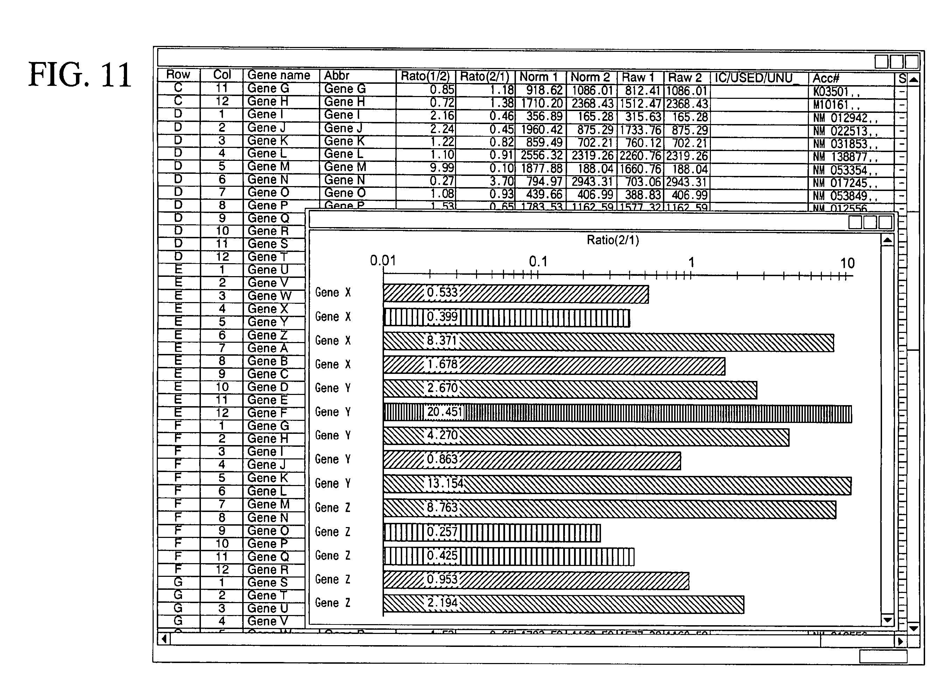

If the measurement information is arranged as shown in FIG 1, it may be difficult to analyze the microarray while comparing information supplied by the bar graph and the table since the bar graph and the table are small. In this case, as shown in FIG 11, the table is displayed across the main window while the bar graph is displayed on another window to analyze the data more easily. More specifically, only the bar graph is displayed in another window, and only numerical data is displayed in an entire area of a main window while the bar graph is displayed in the another window. While the bar graph is displayed on another window, the layout of the main window displaying the table may be adjusted. If another window displaying the bar graph is closed, the main window automatically returns to its original display state. The foregoing operation can be performed by sequentially clicking "display" and "graph display" from a menu.
According to the foregoing examples, the display state is changed or checked by clicking the right button of the mouse on the bar graph to display the window, but not limited thereto. Alternatively, a user may adjust or check the display state from a menu bar or a tool bar.

### Fifth Embodiment

The display method of the analysis result of the microarray may include a method of displaying a change in the amount of nucleic acid over all probe spots. FIG 12 illustrates a polygonal line graph illustrating changes in the amount of nucleic acid. A horizontal axis refers to a position of the probe spots while a vertical axis represents signal intensity or the ratio of nucleic acid with a plot. The graph which is provided in the bottom-left of FIG 12 plots the position of the probe spots in a horizontal axis, and plots the amount ratio of nucleic acid in a vertical axis. The plot which stands out upward or downward, centering on 1, represents a change in the amount of nucleic acid. The graph which is provided in the bottom-right of FIG 12 plots the position of the probe spots in a horizontal axis and plots the signal intensity data in a vertical axis. The data of the two tests samples is plotted in a different shade. Thus, a user may acknowledge that the drastic change occurs in a place where two plots are spaced from each other, and the level of the changed signal intensity may be displayed. As the graphs are displayed on the left and right sides of the screen, a user determines the ratio of nucleic acid and the change in the signal intensity level at a glance, and can analyze the microarray in more detail.

In the graph of the amount ratio of the biologically relevant material and the signal intensity data, it is possible to extract a case where extremely-strange normalization was carried out if a user can select whether to use the raw data or the normalized data.
If a user uses a selection unit, e.g. places a mouse pointer on the plot to display a window including position information of a genomic DNA fragment in a chromosome, a gene name, a probe name and numerical data, he/she can receive detailed information on the plot from the graph.

FIG 12 illustrates the polygonal line graph. If a comprehensive analysis is performed with microarray CGH, the probes which include a starting position to an ending position on chromosomes are spotted. In this case, it means that the respective plots in the horizontal axis in the graph bind to each other through a polygonal line. However, if only part of the chromosomes is to be measured, the polygonal line may not necessarily bind the plots. In consideration of such analysis, the polygonal line may be removed and the plots bind to each other according to a state of the plots or groups. Thus, the display method is preferably selected according to the analysis content. The plurality of correlated probe spots on the microarray can be identified on the replicate image to display the group extracted fragmentarily. For example, the correlated probe spot groups on the replicate image may bind in a circle, or the table may include a group item to display the microarray data more easily.

FIG 12 is displayed only with the polygonal line graph, but a bar graph as shown in FIG 9 may be used to display the cases of being changed and unchanged by distinguishing with colors for a more visible display.

FIG 12 illustrates the polygonal line graph which has items in the horizontal and vertical axes and is arranged as described above. However, the items of the vertical and horizontal axes or the arrangement of the graph are not limited thereto. Alternatively, the display method of the graph may vary as long as it is appropriate to analyze the microarray. The polygonal line graph, the plots and the bar graph are used to visualize the analysis result as an example of the present exemplary embodiment, but not limited thereto. The display method may vary as long as it describes a change in the amount of nucleic acid.

### Sixth Embodiment

A high signal or a very low signal may be detected from probe spots on a microarray according to reacted test samples. For example, if the expression levels of a gene are analyzed, the probe spot at a high signal intensity level represents a gene at a high expression level while the probe spot at a low signal intensity level represents a gene at a low expression level. As the signal intensity of the respective probe spots on the microarray is compared, the values of genes in both high and low expression levels should be calculated precisely. Even if the probe spots at both high and low signal intensity levels are present on the microarray, the respective probe spots may be provided with the optimal exposure conditions. An image of the respective probe spots obtained under the optimal exposure conditions should be displayed.

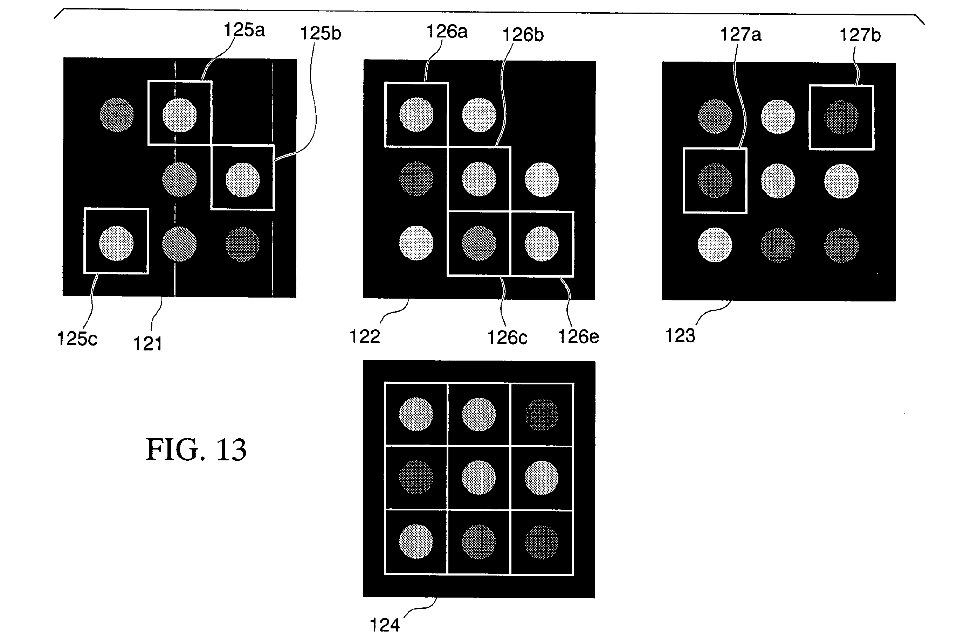

Hereinafter, the signal detecting method will be described with reference to FIG 13. The signal detecting conditions may vary to detect a signal with the optimal probe spots. A method of doubling the exposure time may be suggested. As shown in FIG 13, an image 121 refers to an image generated in one second, an image 122 is an image generated in two seconds and an image 123 is an image generated in four seconds. In the image 121, the probe spots which are supplied under the optimal exposure conditions include 125a, 125b and 125c in a rectangle. In the image 122, the probe spots which are supplied under the optimal exposure conditions include 126a, 126b, 126c and 126d in a rectangle. In the image 123, the probe spots which are supplied under the optimal exposure conditions include 127a and 127b in a rectangle. The signal detecting condition may include, e.g. the exposure time, an optical filter, sensitivity of a detector, intensity of illuminating light, etc.

An image 124 in FIG 13 refers to an image which combines the probe spots extracted from the images generated under the respective exposing condition. The image 121 includes the probe spot which is too dark to be seen and the image 123 includes the probe spot which is too bright. However, the respective images 121, 122 and 123 combine into the image 124 which provides overall probe spots.
As described above, the image which provides the respective probe spots supplied under the optimal exposure conditions can be generated.

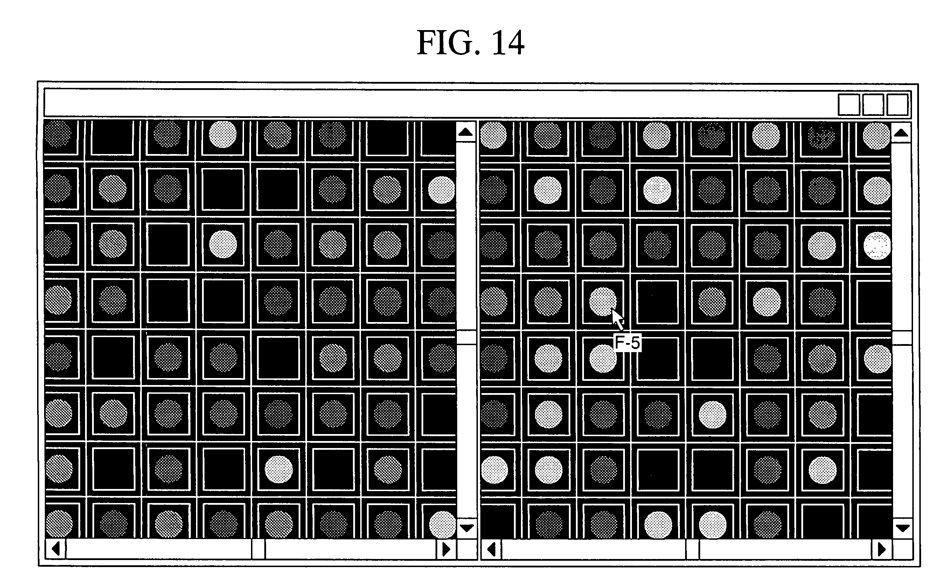

FIG 14 illustrates an example of an image which is provided as an image on a single microarray by combining the images generated under optimal signal detecting conditions with respect to the respective probe spots. Respective cells in matrix pattern represent a region of the probe spots, and the image is combined with the regions of the probe spots with optimal viewing conditions. If an image which is generated under optimal conditions with respect to the bright probe spot is displayed, instead of displaying the image generated under optimal conditions with respect to the respective probe spots, a dark probe spot may not be shown on a screen. Then, it is difficult to analyze the shape and state of the probe spots. However, as show in FIG 14, the images which are generated under the optimal signal detecting condition with respect to the respective probe spots are combined and displayed, thereby displaying the dark probe spot more brightly and enabling a user to analyze the shape and state of the overall probe spots.

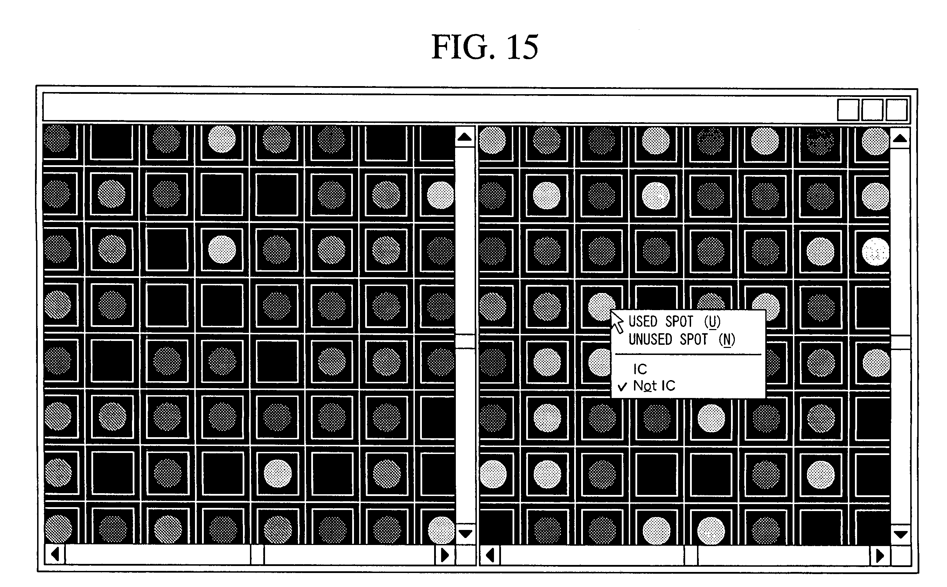

As shown in FIG 14, if a mouse pointer is moved to the region of the probe spots, the position of the probe spot on the microarray is displayed. This is very convenient because a user can recognize the currently-analyzed probe spot quickly.
If it is determined according to the analysis result that some probe spots should not be analyzed since the shape of the probe spots is not circular or impurities exist in the region of the probe spots, a user may click the right button of the mouse on the region of the probe spot to display an adjustment window, and can change the state of the probe spot into an unused probe spot as shown in FIG 15. A user can change whether to include the probe spot as the normalized, biologically relevant material. A user may change the state of the probe spots while analyzing the microarray data with the displayed image, thereby allowing the analysis of the microarray data precisely and quickly.

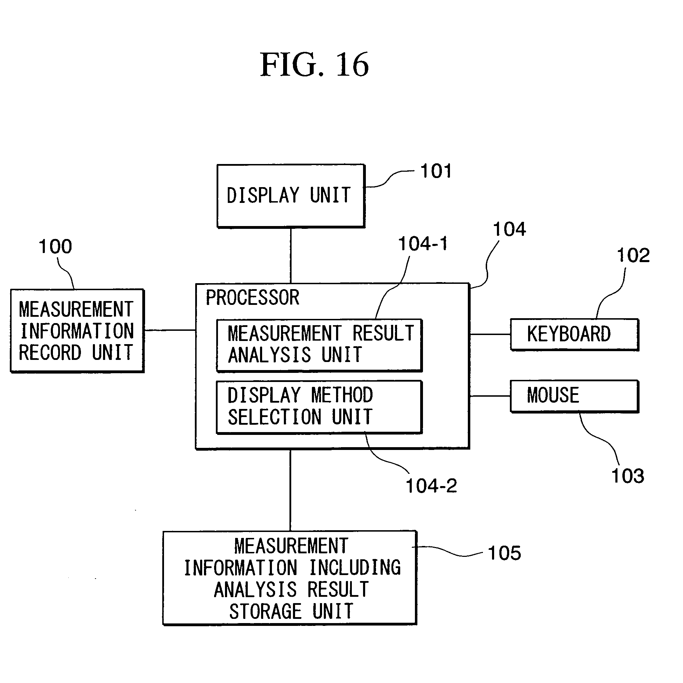

FIG 16 is a block diagram of a system according to the display method of the present invention. The system includes a measurement information record unit 100 which records measurement information including bibliographic facts such as a measured object, the position of the probe spot, the serial number of the microarray, the name of the experimenter, measurement date and place, the type of test samples, measurement conditions such as a measuring device, a measurement method, the type of the optical filter, the measurement temperature, the pH, the measurement time, the measurement result such as the signal intensity data; a display unit 101 which visually displays measurement information including the analysis result analyzing the measurement result such as data normalization, the amount of the biologically relevant material or the amount ratio of the biologically relevant material among the plurality of test samples, other than the bibliographic facts, the measurement conditions and the measurement result; a keyboard 102 which is provided for input, selection and manipulation of values from the system; a mouse 103; a processor 104 which has a measurement result analysis unit 104-1 which analyzes the measurement result; and a display method selection unit 104-2 which is provided to select a display method of the signal intensity data in different shades, a display method of the probe spots, a display method of the shape of the probe spots and the measurement information by placing the mouse pointer on the probe spots, a display method of the replicate image, the table, the ratio diagram and the distribution diagram, a display method of the normalization method, a display method of the bar graph, the polygonal line graph and the plots with respect to the amount ratio of the biologically relevant material, a parallel display method of the images, a setting range of the amount ratio of the biologically relevant material to be displayed, a display method of the graphs, a display method of the image according to the proper signal detecting condition, etc.; and a measurement information storage unit 105 which includes a folder storing the measurement information having the analysis result.

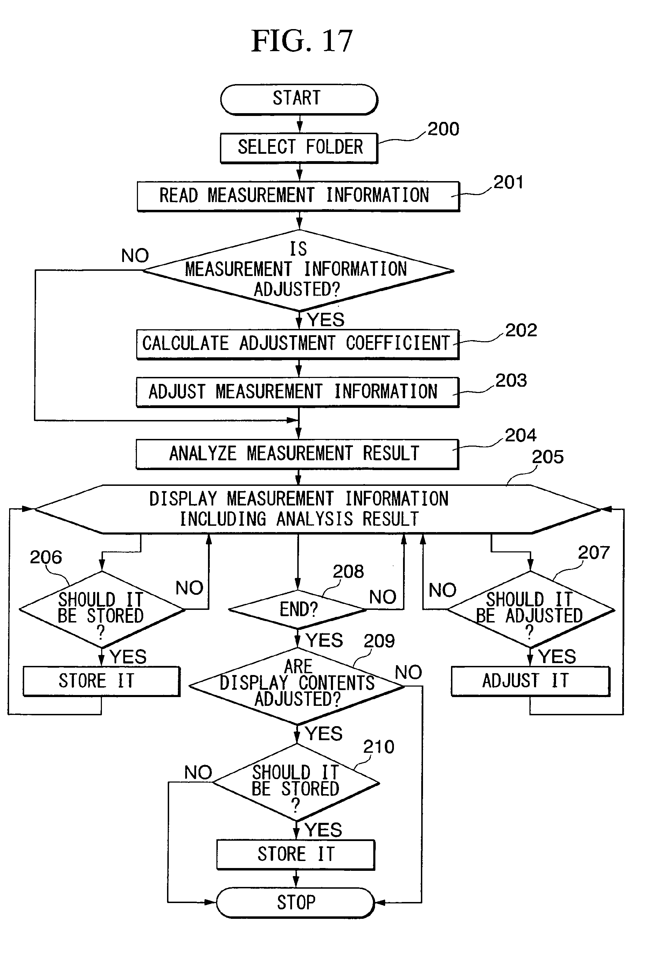

FIGS. 17 to 21 illustrate schematic control flowcharts according to the display method of the invention.
FIG 17 is a schematic flowchart of the invention.
First, a user selects a folder which stores the measurement information to be analyzed (step 200). Then, the measurement information having the analysis result is displayed on the screen (step 205). If a user desires to change the display content (step 207), they can select the content to be changed. Then, a user completes the analysis (step 208) by making a selection, thereby storing the analysis result.
Next, the order of operation including a process in the software is that a user first selects the folder storing the measurement information having the analysis result (step 200), then the processor 104 reads the measurement information of the particular test sample from the storage unit 100 (step 201). At step 202, the measurement result analysis unit 104-1 calculates the normalization coefficients based on the signal intensity raw data. Then, the data is normalized at step 203. After the foregoing operations are completed, the measurement result analysis unit 104-1 analyzes the measurement result such as calculating the amount of nucleic acid or the amount ratio of nucleic acid based on the signal intensity data at step 204. The display unit 101 displays the measurement information including the analysis result thereon at step 205. If a user inputs a command to store the measurement information including the analysis result of the measurement result at step 206, the processor 104 stores the measurement information including the analysis result in a predetermined folder of the measurement information storage unit 105. If a user inputs a command to change the display method at step 207 after the analysis result is displayed, the display method selection unit 104-2 changes the display method according to a user's command. The display unit 101 displays the measurement information including the analysis result reflecting a change. If a user inputs a command to finish the analysis at step 208, the processor 104 determines whether the analysis result is stored. If the analysis result is stored, a user checks whether the display method is changed. If it is determined that the analysis result is not stored or the analysis result is not stored after the displayed method is changed, the processor 104 determines whether to store the measurement information including the analysis result of the measurement result at step 210. If a user desires to store the measurement information and inputs a command, the processor 104 stores the measurement information including the analysis result in the predetermined folder of the measurement information storage unit 105, thereby completing the storage operation.
FIG 17 illustrates a flowchart in which the normalization of the data is performed. However, if there is not much difference in the RNA amount included in the test samples obtained from the cells to be compared, the normalization is not necessary. In this case, steps 202 and 203 are skipped. The amount of nucleic acid or the amount ratio thereof may not be calculated at step 204. The measurement information including the analysis result is stored in the predetermined folder. However, if a user inputs a command to store the measurement information, for example, the measurement information may be automatically stored in the folder that is selected initially.

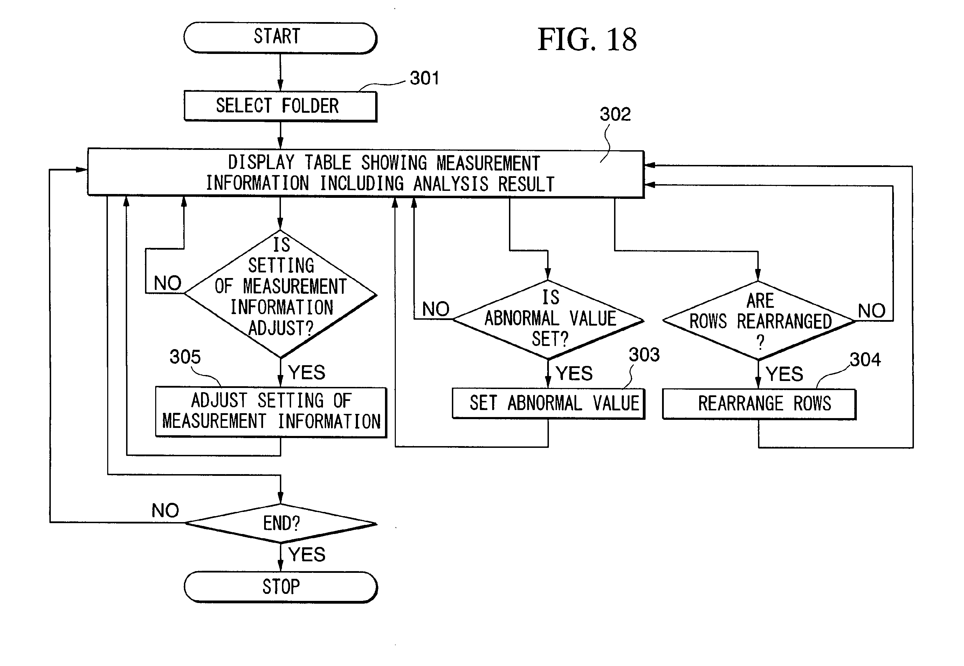

FIGS. 18 to 21 are detailed examples of the flowchart in FIG 17. FIGS. 18 to 21 do not provide operations such as reading the measurement information, calculating the normalization coefficients, the normalization and analyzing the measurement result for purposes of convenience.
FIG 18 is a detailed control flowchart illustratingan operation of displaying the measurement information including the analysis result obtained in FIG 17 if a user enters a command to change the table displaying the measurement information. If the folder which stores the measurement information including the analysis result of the test samples to be displayed is selected (step 301), the measurement information including the analysis result of the test samples is displayed (refer to FIG 1). The display unit displays the bibliographic facts, the measurement result, the analysis result table, the replicate image, the bar graph illustrating the amount ratio of the biologically relevant material, the distribution diagram, the folder name, the normalization method and normalization coefficients. To set an abnormal value (probe spot not used in calculating the normalization coefficients), a user may sequentially click "setup", "normalization method" and "setting threshold value" items from the menu. If the command to set the abnormal value is input, the measurement result analysis unit 104-1 sets the abnormal value (step 303). If the measurement result analysis unit 104-1 detects the abnormal value, the mark is placed on the right of the cell of the corresponding probe spot. The display method selection unit 104-2 displays the selected data in blue, the normalized, biologically relevant material, e.g. the probe spot of the internal control gene in red, and the unused data in light blue.
The table can be rearranged according to ascending or descending order by clicking a reference row. The rows can be rearranged by dragging the items. The table may be arranged by clicking the reference row or dragging the items. If a user inputs a command to rearrange the table, the display method selection unit 104-2 rearranges the table (step 304). A user can select whether to use the data or not and whether to use the data for the normalization or not. This setting may be changed by clicking the right button of the mouse on the selected row, displaying the selection box and clicking the corresponding item therefrom. Then, the command to change the setting is input, and the display method selection unit 104-2 changes the setting (step 305).
A user can set whether to use the data by setting the threshold value. A user may sequentially click "set up" and "analysis condition" items from the menu and input the threshold value with respect to the displayed window to set the threshold value. Then, the command to set the threshold value is input, and the measurement result analysis unit 104-1 sets the threshold value (step 303).

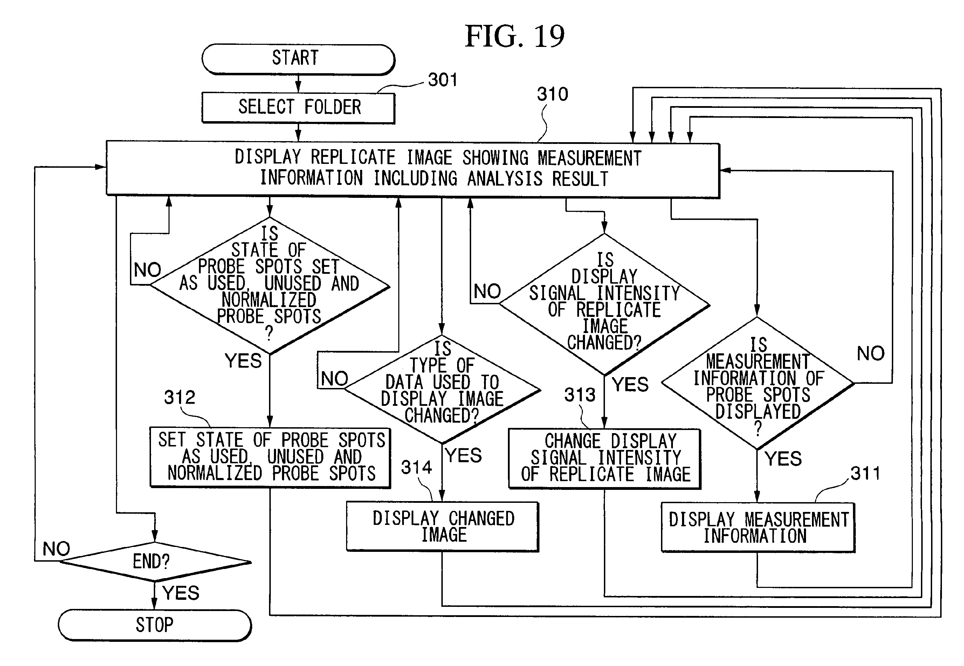

FIG 19 is a schematic control flowchart of the replicate image displaying the measurement information including the analysis result obtained in FIG 17. The display method selection unit 104-2 displays the replicate image of the test sample 1 in red, the replicate image of the test sample 2 in green and the combined replicate image of the test samples 1 and 2 in a color mixed with red and green. The normalized, biologically relevant material, e.g. the probe spot of the internal control gene is displayed with the red circle, the currently-selected probe spot is displayed with the blue circle and the unused probe spot is displayed with X. The measurement information such as the gene name or the signal intensity of the probe spots can be displayed on the replicate image. The measurement information is displayed by placing the mouse pointer on the probe spot. Then, the command to display the measurement information is input, and the display method selection unit 104-2 displays the measurement information (step 311). The state of the probe spots, i.e. whether the probe spots are currently used, used or unused may be changed by clicking the right button of the mouse on the selected probe spot, displaying the selection box and clicking the corresponding item therefrom. Then, the command to change the state of the probe spot is input, and the display method selection unit 104-2 changes the state of the probe spot (step 312).
The type of the replicate image is changed by sequentially clicking "display" and "replicate image" items from the menu and clicking the desired replicate image (the replicate image of test sample 1, the replicate image of test sample 2 and the combined replicate image of test samples 1 and 2). Then, the command to display the replicate image is input, and the display method selection unit 104-2 displays the replicate image. The display signal intensity of the replicate image is changed by clicking "display setting" item. The command to change the display signal intensity is input, and the display method selection unit 104-2 changes the display signal intensity (step 313). The type of the data used to display the analysis result is changed by adjusting the ON/OFF state of the raw data display. The command to display the changed image is input, and the display method selection unit 104-2 displays the changed image (step 314).

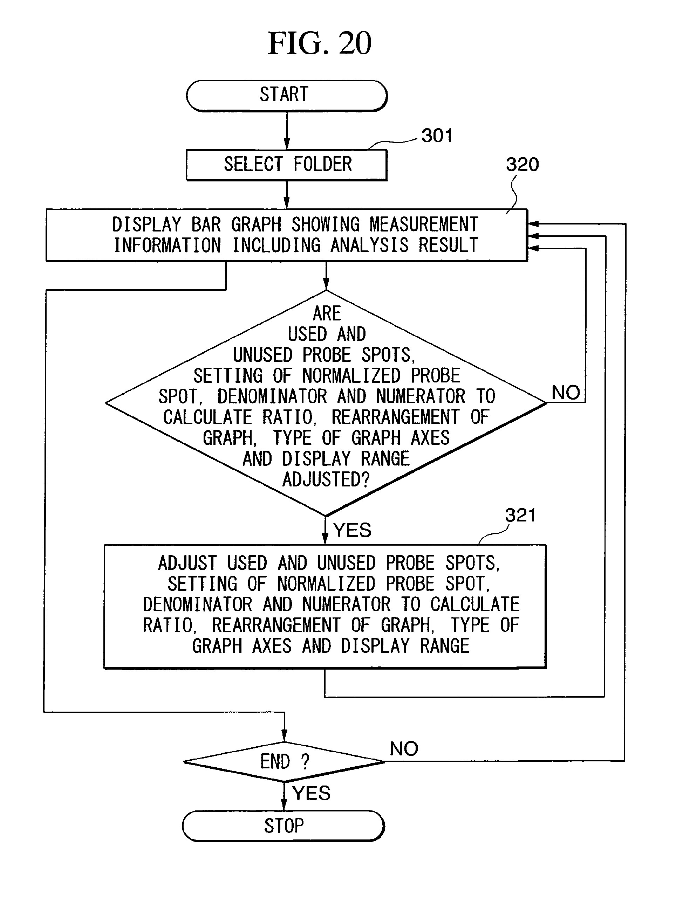

FIG 20 is a schematic control flowchart of the bar graph (ratio diagram) displaying the measurement information including the analysis result obtained in FIG 17. The display method selection unit 104-2 displays the bar graph in red if the ratio between the test sample and the compared object (e.g. standard test sample, a test sample of a healthy person if the experiment is implemented to determine a disease) is 0.5 or below, displays the bar graph in yellow if the ratio is larger than 0.5 and smaller than 2.0, displays the bar graph in green if the ratio is 2.0 and above, and displays the measured probe spot in blue. A user can select whether to use the probe spots for normalization or use the probe spots for the analysis. By comparing reversely, the denominator and numerator may be interchanged to calculate the ratio of nucleic acid. The bar graph can be rearranged according to the gene sequence, the expression level sequence, etc. The type of the axes of the graph, i.e. the linear scale and the log scale can be changed. The display range can be also changed by clicking the right button of the mouse on the graph, displaying the selection box and clicking the concerned item therefrom. Then, the command to change the foregoing elements is input, and the display method selection unit 104-2 changes the foregoing elements.
The items "display" and "ratio diagram" are sequentially clicked from the menu to select the test sample as the denominator, to set the sequence of the bar graph and scales of the vertical and horizontal axes (e.g. setting the ON/OFF state to display a logarithmic axis and inputting values of the display range from the window of the display setting). Then, the command to change the foregoing elements is input, and the display method selection unit 104-2 changes the foregoing elements.

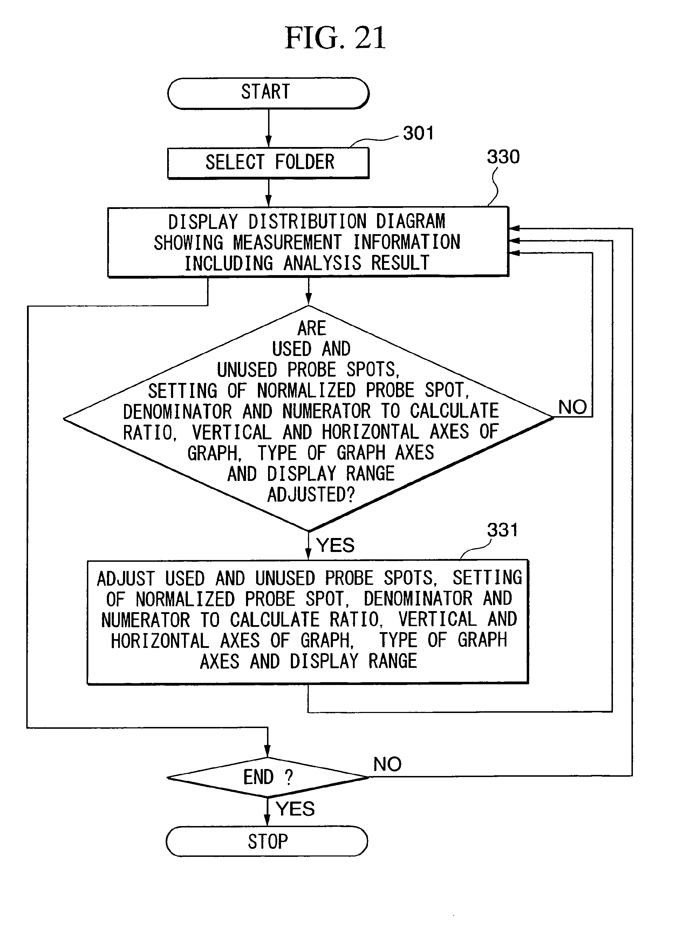

FIG 21 is a schematic control flowchart of the distribution diagram displaying the measurement information including the analysis result obtained in FIG 17. The display method selection unit 104-2 displays the normalized probe spot in red and displays the analyzed probe spot in blue. The change of the probe spot to be used for analysis or not, the change of the normalized probe spot, the interchange between the vertical and horizontal axes and the type of the graph axes can be changed by clicking the right button of the mouse on the graph, displaying the selection box and clicking the concerned item therefrom. Thus, the command to change the foregoing items is input, and the display method selection unit 104-2 changes the concerned items. The drawing range of the graph may be set by clicking the item "display setting." The items "display" and "distribution diagram" are sequentially clicked, and then the items "vertical and horizontal axes adjustment", "logarithmic axis display" or "display setting" can be selected. Then, the command to set the foregoing elements is input, and the display method selection unit 104-2 sets the foregoing elements.

A hybrid image which combines the images of the respective probe spots under the optimal signal detecting condition with respect to the two test samples can be displayed by sequentially clicking the items "display" and "image display" from the menu. Then, the command to display the image is input, and the display method selection unit 104-2 displays the hybrid image.

The bibliographic facts, the measurement conditions and so on may be displayed by sequentially clicking the items "display" and "experiment information display" from the menu. Then, the command to display the experiment information is input, and the display method selection unit 104-2 displays the experiment information.

According to the present exemplary embodiment, the method of displaying the analysis result of nucleic acid as the biologically relevant material by using the microarray is provided. If the test results of multiple items are displayed, the detection method is not limited to the microarray. The display method can be applicable to display the analysis result of other biologically relevant materials such as hormones, a tumor marker, an enzyme, an antibody, an antigen, an abzyme, other proteins, a nucleic acid, cDNA, DNA, mRNA, etc. Particularly, the method is effective for preparation, analysis, and display of a microarray.

### INDUSTRIAL APPLICABILITY

The invention is useful in a technical field detecting a biologically relevant material, particularly plural genes or expression of plural genes in plural biological samples.

## Claims

1. A display method for displaying measurement information of a biologically relevant material, in which the amount of a biologically relevant material of a test sample or the amount ratio of the biologically relevant material among a plurality of test samples is measured, the display method comprising:
displaying measurement information on a computer screen.

2. The display method according to Claim 1, further comprising displaying a replicate image representing a layout of probe spots on a microarray as the measurement information.

3. The display method according to Claim 2, wherein the replicate image is normalized and displayed in 256 gradation levels and a maximum value of signal intensity of overall measured probe spots is normalized and displayed in 256 gradation levels.

4. The display method according to Claim 2, wherein the replicate image is normalized and displayed in 256 gradation levels and a predetermined signal intensity is normalized and displayed in 256 gradation levels.

5. The display method according to Claim 2, wherein:
the replicate image is displayed in a different color for a different test sample;
the replicate image is displayed in mixed colors if measurement information of respective test samples are displayed with overlapping each other; and
the amount of the biologically relevant material and/or the amount ratio of the biologically relevant material among the plurality of test samples is displayed in different shades.

6. The display method according to Claim 5, further comprising displaying a probe spot based on raw data of signal intensity of the probe spot.

7. The display method according to Claim 5, further comprising displaying a probe spot based on normalized signal intensity data of the probe spot.

8. The display method according to Claim 5, further comprising displaying a probe spot based on either one of raw data of signal intensity of the probe spot or normalized signal intensity of the probe spot which is displayed alternately.

9. The display method according to Claim 1, further comprising displaying the amount of the biologically relevant material in 256 shades.

10. The display method according to Claim 2, wherein an image of each replicate probe spot is displayed on the replicate image in a circle whose color density is higher at the center and gradually becomes lower toward the circumference thereof.

11. The display method according to Claim 2, wherein the replicate image also displays information of position of each probe spot on the microarray.

12. The display method according to Claim 2, further comprising displaying on the replicate image a first mark on a selected replicate probe spot, a second mark on a replicate probe spot that has been omitted from the analysis, a third mark on a probe spot corresponding to a gene that is used for normalization and no mark on probe spots which are not included in any of 3 conditions described foregoing and are used for analysis.

13. The display method according to Claim 12, wherein the used and unused probe spots can be selected and/or changed with any one of a replicate image, a table, a ratio diagram and a distribution diagram, and the replicate image is adjusted according to the selected and/or changed probe spots.

14. The display method according to Claim 12, wherein the probe spot for normalization can be selected and/or changed with any one of the replicate image, the table, the ratio diagram and the distribution diagram, and the replicate image is adjusted whenever the probe spot for normalization is selected and/or changed.

15. The display method according to Claim 2, further comprising displaying the measurement information by placing a selection means on the probe spot of the replicate image.

16. The display method according to Claim 15, wherein the measurement information comprises a gene name corresponding to the probe spot.

17. The display method according to Claim 15, wherein the measurement information comprises the raw data of the signal intensity of the probe spot.

18. The display method according to Claim 15, wherein the measurement information comprises normalized signal intensity of the probe spot.

19. The display method according to Claim 1, further comprising measuring the amount of the biologically relevant material of a test sample using the microarray and normalizing the obtained biologically relevant material to compare the amount of the biologically relevant material among the respective test samples.

20. The display method according to Claim 19, wherein normalization of the amount of the biologically relevant material is preformed by using a gene for normalization.

21. The display method according to Claim 20, further comprising displaying the gene used for normalization in at least one of the replicate image, the table, the ratio diagram and the distribution diagram.

22. The display method according to Claim 20 or 21, wherein the gene used for normalization is adjustable.

23. The display method according to Claim 22, wherein the gene for normalization can be changed by using any one of the replicate image, the table, the ratio diagram and the distribution diagram, and the changed state of the gene for normalization is displayed on a window.

24. The display method according to Claim 19, wherein the normalization of the amount of the biologically relevant material is performed by using an average of signal intensities of overall probe spots.

25. The display method according to Claim 19, wherein the normalization of the amount of the biologically relevant material is preformed by using a median of the signal intensities of overall probe spots.

26. The display method according to Claim 19, further comprising displaying a currently-used normalization method.

27. The display method according to Claim 19, further comprising displaying a coefficient value which is used to normalize the amount of the biologically relevant material.

28. The display method according to Claim 1, wherein the measurement information comprises information of a measurement condition.

29. The display method according to Claim 1, wherein the measurement information comprises information of a folder or the name of the folder that contains the measurement information including the analysis result.

30. The display method according to Claim 1, wherein the measurement information comprises the name of the test samples that have been analyzed.

31. The display method according to Claim 1, wherein the measurement information comprises a serial number of the microarray that has been used.

32. The display method according to Claim 1, wherein the measurement information comprises the name of the person who performed the experiment.

33. The display method according to Claim 1, wherein the measurement information comprises the type of used exciting and absorbing optical filter.

34. The display method according to Claim 1, wherein the measurement information comprises the information of temperature during the experiment.

35. The display method according to Claim 1, wherein the measurement information comprises the amount of the biologically relevant material and/or the amount ratio of the biologically relevant material among a plurality of test samples.

36. The display method according to Claim 1, further comprising displaying the amount ratio of the biologically relevant material between two test samples to be compared, with a bar graph (ratio diagram).

37. The display method according to Claim 36, further comprising varying colors of the bar graph according to the amount ratio of the biologically relevant material, and displaying a bar graph of the currently-selected probe spot in another color.

38. The display method according to Claim 36, wherein the order of samples displayed in bar graph can be changed by selecting the ordering methods either by the gene name or by the ratio amount of biologically relevant material to arrange the bar graph.

39. The display method according to Claim 36, wherein the setting of the display range of the amount ratio of the biologically relevant material can be changed.

40. The display method according to Claim 36, further comprising interchanging a denominator and a numerator to calculate the amount ratio of the biologically relevant material to be compared.

41. The display method according to Claim 36, further comprising displaying only the bar graph in another window, and displaying only numerical data in an entire area of a main window while the bar graph is displayed in the another window.

42. The display method according to Claim 1, further comprising displaying a relationship between a position of a probe spot and the amount ratio or the signal intensity of the biologically relevant material between two test samples to be compared, with respect to the plurality of probe spots on the microarray, with a polygonal line graph or a plot.

43. The display method according to Claim 42, further comprising displaying the position information of a genomic DNA fragment on a chromosome, a gene name, a probe name and numerical data in respective plots of the polygonal line graph or the plots.

44. The display method according to Claim 42, wherein the amount ratio or the signal intensity of the two biologically relevant materials to be compared is displayed based on the raw data of signal intensity or the normalized signal intensity of the probe spots.

45. The display method according to Claim 42, further comprising displaying a graph representing a relationship between the position of the probe spot and the amount ratio of the biologically relevant material of the two test samples to be compared, in parallel with a graph representing a relationship between the position of the probe spot and the signal intensity of the biologically relevant material of the two test samples to be compared.

46. The display method according to Claim 42, further comprising displaying a plurality of correlated probe spots on the microarray so as to be distinguished from the other probe spots on the same microarray in the replicate image.

47. The display method according to Claim 1, further comprising displaying an combined image of each probe spot in which the each image of the each probe spot is generated under the optimal signal detecting condition for each probe spot respectively.

48. The display method according to Claim 47, wherein the combined respective images are displayed to correspond to the order of the probe spots on the microarray.

49. The display method according to Claim 47 or 48, wherein the state of the probe spots as used, unused or as used for the normalization can be adjusted.

50. The display method according to Claim 1, further comprising displaying measurement information including a replicate image of probe spots of microarray, a bar graph, a distribution diagram and numerical data on the same window simultaneously.

51. The display method according to Claim 1, further comprising displaying measurement information including a replicate image of probe spots of microarray, numerical data, a polygonal line graph and a plot on the same window simultaneously.
